# EUROPÄISCHE PATENTANMELDUNG

(11) **EP 2 055 396 A2**
(43) Veröffentlichungstag der Anmeldung: **06.05.2009**
(21) Anmeldenummer: 08018783.4
(22) Anmeldetag: 28.10.2008
(51) Int. Cl.: B08B 7/00

(54) **Reinigungsvorrichtung zur Reinigung von insbesondere mit Algen oder Moos befallenen flächigen Gegenständen sowie Reinigungsverfahren mit einer derartigen Reinigungsvorrichtung**

(30) Priorität: 03.11.2007 DE 102007052566
(71) Anmelder: h & m gutberlet gmbh, 90471 Nürnberg (DE)
(72) Erfinder: Gutberlet, Michael, 90471 Nürnberg (DE)
(74) Vertreter: Hofmann, Matthias

(57) **Zusammenfassung**

Eine Reinigungsvorrichtung (1) dient zur Reinigung von insbesondere mit Algen oder Moos (2) befallenen flächigen Gegenständen (3). Teil der Reinigungsvorrichtung (1) ist eine flächige Habitat-Vorgabeeiorichtung (4), welche zur Schaffung eines Lebensraums (5) für Mikroorganismen auf den zu reinigenden Gegenstand (3) diesen abdeckend aufbringbar ist Eine derartige Reinigungsvorrichtung sowie ein Reinigungsverfahren hierfür gewährleisten eine schonende und wenig aufwändige Reinigung.

## Beschreibung

Die Erfindung betrifft eine Reinigungsvorrichtung zur Reinigung von insbesondere mit Algen oder Moos befallenen flächigen Gegenständen. Ferner betrifft die Erfindung ein Reinigungsverfahren mit einer derartigen Reinigungsvorrichtung.

Derartige Reinigungsvorrichtungen sind, beispielsweise in Form von Dampfstrahlern, durch offenkundige Vorbenutzung bekannt. Andere bekannte Reinigungsvorrichtungen sind Sandstrahl-Vorrichtungen oder chemische Reinigungsmittel. Nachteilig ist, dass bei den bekannten Reinigungsvorrichtungen die Struktur des zu reinigenden Gegenstandes angegriffen und beschädigt werden kann. Zudem sind die bekannten Reinigungsvorrichtungen in ihrer Anwendung aufwändig.

Aus der DE 696 3 5 045 T2 sind Zusammensetzungen und Verfahren zur Behandlung von Wasserlebewesen oder terrestrischen Organismen bekannt.

Aus der DE 103 48 732 A1 ist ein Verfahren zur Reinigung von Wasser offenbart.

Die US 5 998 200 offenbart Verfahren und Zusammensetzungen zur Behandlung von Flächen unter Wasser, um den Bewuchs mit aquatischen Organismen zu verhindern.

Die GB 2 326 114 A bezieht sich auf einen Wasserbehandlungs-Behälter mit einem Bett aus partikulärem Material, wobei die Partikel des partikulären Materials ein Habitat für Mikroorganismen bilden, die bei der Abwasserbehandlung wirksam sind.

In der DE 24 20 927 A1 ist eine Schutzanordnung gegen den schädlichen Bewuchs von Unterwasser-Stahlbauteilen offenbart.

Die DE 27 39 376 A1 offenbart Antifouling-Schutzmittel, die den Anwuchs von Meeresorganismen an Schiffsböden und Unterwasserbauten verhindern.

Die DE 29 00 505 A1 offenbart einen als Geflecht oder Gitter ausgeführten Belag für einen Gegenstand, wobei der Belag einen Träger aus Kunststoff aufweist, der Kupfer oder eine Kupferlegierung enthält.

Es ist eine Aufgabe der vorliegenden Erfindung, eine Reinigungsvorrich-tung der eingangs genannten Art und ein Reinigungsverfahren hierfür derart weiterzubilden, dass eine schonende und wenig aufwändige Reinigung möglich ist.

Diese Aufgabe ist erfindungsgemäß gelöst mit einer Reinigungsvorrichtung mit den im Anspruch 1 angegebenen Merkmalen.

Erfindungsgemäß wurde erkannt, dass eine Reinigung flächiger Gegenstände, die mit Algen oder Moos befallen sind, auf überraschend einfache Weise möglich ist. Es reicht aus, in den zu reinigenden Bereichen der Gegenstände einen Lebensraum, also ein Habitat, für die Verschmutzung des Gegenstandes abbauende Mikroorganismen bereit zu stellen. Hierzu dient die Habitat-Vorgabeeinrichtung. Durch die Abdeckung zumindest der zu reinigenden Bereiche des zu reinigenden Gegenstandes wird zwischen der Habitat-Vorgabeeinrichtung und dem Gegenstand der Lebensraum für die Mikroorganismen gebildet. Diese können sich beispielsweise auf natürlichem Wege ansammeln und vermehren sich, solange sie beispielsweise Nahrung in der biologisch abzubauenden Verschmutzung finden. Auf diese Weise bauen die Mikroorganismen die Verschmutzung ab, bis diese vollständig verschwunden ist. Die darunter liegende Struktur des Gegenstandes wird von den Mikroorganismen nicht angegriffen. Als Mikroorganismen können Bakterien-, Pilz- oder Zellkulturen zum Einsatz kommen. Der mikrobiologische Abbau kann durch jegliche Bioreaktion, insbesondere durch Fermentation erfolgen. Der Reinigungsaufwand beschränkt sich auf die Auftragung und gegebenenfalls noch auf das Entfernen der Habitat-Vorgabeeinrichtung.

Bei einer Habitat-Vorgabeeinrichtung nach Anspruch 2 kann in vielen Fällen sogar der dem Reinigungsvorgang nachfolgende Schritt des Ablösens der Habitat-Vorgabeeinrichtung eingespart werden. Die Habitat-Vorgabeeinrichtung löst sich beispielsweise während eines Regenschauers auf.

Bei einer Ausgestaltung nach den Ansprüchen 3 und 4 lässt sich je nach dem UV-Absorptionsgrad und/oder der Luftdurchlässigkeit der Habitat-Vorgabeeinrichtung ein Populationswachstum der Mikroorganismen steuern. Die Habitat-Vorgabeeinrichtung kann auch so ausgebildet sein, dass sie ein Volumen, welches die zu reinigende Oberfläche des zu reinigenden Gegenstandes beinhaltet, luftdicht abschließt. In diesem Fall kann der Reinigungsvorgang so ablaufen, dass die biologisch abzubauende Verschmutzung, beispielsweise Algen, Moos, Flechten usw., wegen fehlender Sauerstoffzufuhr abstirbt und von den Mikroorganismen abgebaut wird. Ein derartiges Absterben kann auch durch eine Lichtundurchlässigkeit der Habitat-Vorgabeeinrichtung bewirkt werden.

Bei einer Habitat-Vorgabeeinrichtung nach Anspruch 5 kann der Wärmehaushalt zwischen dieser und dem zu reinigenden Gegenstand vorgegeben und ein optimales Wachstumsklima für die Mikroorganismen erzeugt werden. Die IR-Durchlässigkeit wird dabei auf die einzusetzenden Mikroorganismen sowie auf den Typ des zu reinigenden Gegenstandes abgestimmt. Eine zusätzlich gegebenenfalls erforderliche absorbierende Wirkung im sichtbaren Spektralbereich (VIS) kann durch gezielte Einfärbung der Habitat-Vorgabeeinrichtung fein beeinflusst werden.

Ein Reservoir nach Anspruch 6 gewährleistet eine definierte Anfangsmenge von bei der Reinigung einzusetzenden Mikroorganismen. Je nach den Umgebungsbedingungen bei der Reinigung kann auf ein derartiges Reservoir auch verzichtet werden. In diesem Fall wird eine natürlich vorhandene Mikroorganismen-Anfangsmenge eingesetzt. Zwischen dem Reservoir und beispielsweise einem Abdeckmedium der Habitat-Vorgabeeinrichtung kann eine weitere Schicht mit einer Trennwand oder einer Trennschicht vorgesehen sein, wobei diese Trennwand oder Trennschicht durch gezielt beaufschlagte Einflüsse oder durch Umwelteinflüsse, beispielsweise durch Druck oder Temperatur, zum Kollabieren gebracht werden kann, sodass die Mikroorganismen frei werden und die Reinigung beginnen kann. Als Mikroorganismus kann insbesondere das Bakterium Bacillus Natto eingesetzt werden, das im Zusammenhang mit der Herstellung eines japanischen Lebensmittels aus vergorenen Sojabohnen bekannt ist.

Eine nährstoffhaltige Habitat-Vorgabeeinrichtung nach Anspruch 7 stellt definierte Wachstumsbedingungen für die Mikroorganismen bereit.

Eine nährstoffhaltige Beschichtung nach Anspruch 8 ist eine unaufwändige Variante zur Bereitstellung von Nährstoffen. Alternativ können die Nährstoffe einem Grundmedium, welches die Habitat-Vorgabeeinrichtung aufbaut, auch beigemischt werden.

Eine Folie nach Anspruch 9 lässt sich mit geringem Aufwand auf den zu reinigenden Gegenstand aufbringen. Hierbei kann auch eine selbstklebende, selbsthaftende, insbesondere aufgrund elektrostatischer Effekte haftende Folie eingesetzt werden.

Eine Beschichtung nach Anspruch 10 lässt sich ebenfalls mit geringem Aufwand auftragen.

Dies gilt insbesondere für ein Anstrichmittel nach Anspruch 11. Als Anstrichmittel können beispielsweise ein Lack, eine Farbe, insbesondere eine Leimfarbe, eingesetzt werden.

Die Habitat-Vorgabeeinrichtung kann als Schaum ausgeführt sein. Ein derartiger Schaum eignet sich insbesondere für schwer zugängliche und/oder verwinkelte Bauelemente.

Eine Fasermaterial-Abdeckung nach Anspruch 12 kann bahnweise oder stückweise mit geringem Aufwand aufgelegt oder angebracht werden.

Eine knetbare Abdeckmasse nach Anspruch 13 eignet sich insbesondere für empfindliche und/oder verwinkelte und/oder zierliche zu reinigende Gegenstände.

Die Vorteile des Reinigungsverfahrens nach den Ansprüchen 14 und 15 entsprechen denen, die vorstehend unter Bezugnahme auf die erfindungsgemäße Reinigungsvorrichtung schon erläutert wurden.

Nach erfolgter Reinigung durch die Mikroorganismen, deren Lebensraum zwischen der Habitat-Vorgabeeinrichtung und dem zu reinigenden Gegenstand gebildet ist, kann die Habitat-Vorgabeeinrichtung vom Gegenstand abgelöst werden. Dies erfolgt insbesondere dann, wenn sich die Habitat-Vorgabeeinrichtung nicht nach Ablauf einer bestimmten Zeitdauer von selbst zersetzt.

Ausführungsbeispiele der Erfindung werden nachfolgend anhand der Zeichnung näher erläutert. In dieser zeigen:
- Fig. 1: im Vertikalschnitt einen zu reinigenden Bodenbereich mit einer Reinigungsvorrichtung;
- Fig. 2 bis 4: in einer zu Fig. 1 ähnlichen Darstellung weitere Ausführungen von Reinigungsvorrichtungen; und
- Fig. 5: im Querschnitt einen Abschnitt eines Zierelements aus Stein mit einer weiteren Ausführung einer Reinigungsvorrichtung.

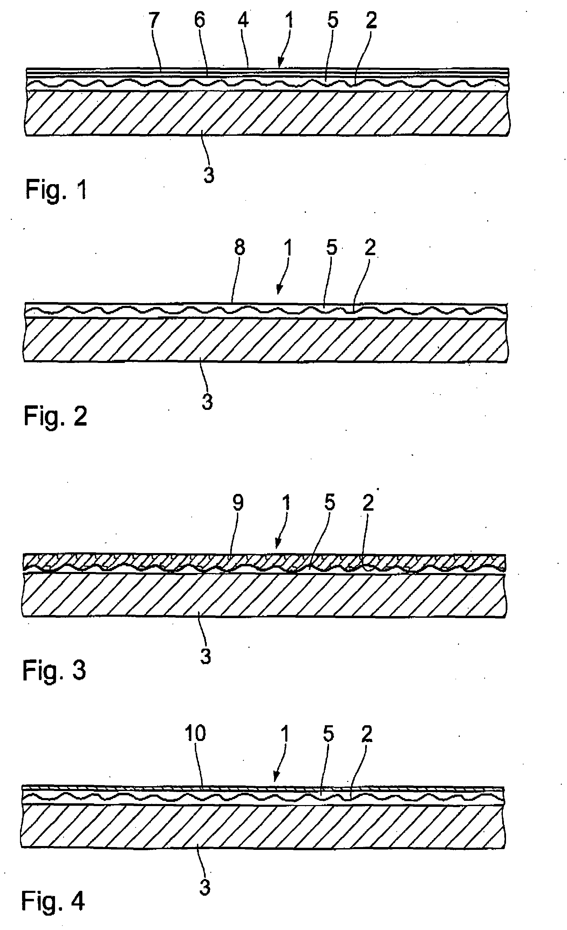

Eine Reinigungsvorrichtung 1, von der in der Fig. 1 eine erste Ausführung dargestellt ist, dient zur Reinigung von insbesondere mit Algen oder Moos 2 befallenen Gegenständen. Dies ist in der Fig. 2 am Beispiel einer steinernen Terrassenplatte 3 als zu reinigendem Gegenstand dargestellt. Die Reinigungsvorrichtung 1 kann auch bei beliebigen anderen flächigen Gegenständen eingesetzt werden, insbesondere an Gebäudefassaden oder an sonstigen Elementen, die mit einer biologisch abbaubaren Patina versehen sind. Beim zu reinigenden Gegenstand kann es sich beispielsweise auch um ein Dach oder um Zierelemente, insbesondere aus Stein, handeln.

Bei der Ausführung nach Fig. 1 ist eine Folie 4 Bestandteil einer flächigen Habitat-Vorgabeeinrichtung für Mikroorganismen. Die Folie 4 dient also zur Schaffung eines Lebensraums für Mikroorganismen in einem Volumen 5 zwischen der Folie 4 und der Terrassenplatte 3. Bei den Mikroorganismen handelt es sich insbesondere um Bakterienkulturen, die die Algen und das Moos 2 biologisch abbauen.

Die Folie 4 wird in an sich dem Fachmann bekannter Weise auf der Terrassenplatte 3 angebracht, sodass die Terrassenplatte 3 durch die Folie 4 flächig abgedeckt ist. Diese Anbringung kann beispielsweise mittels randseitiger Klebebänder erfolgen. Bei der Folie 4 kann es sich auch um eine selbstklebende, um eine selbsthaftende, insbesondere um eine zur Haftung an der Terrassenplatte 3 statisch aufgeladene Folie handeln. Die Folie 4 ist vorzugsweise aus Polypropylen (PP), vorzugsweise orientiert (OPP), oder aus Castpolypropylen (CPP), also gegossenem Polypropylen, Polyethylen (PE), Polyester oder aus einem Polyactid (PLA).

Die Folie 4 ist insbesondere aus einem Material, das biologisch abbaubar ist. Insbesondere kann die Folie 4 aus einem wasserlöslichen Material ausgeführt sein. Das Material der Folie 4 sowie dessen Schichtdicke sind so aufeinander abgestimmt, dass die Folie 4 aus einem für UV-Strahlung und IR-Strahlung teilweise absorbierenden Material ausgeführt ist.

Die Folie 4 ist durch eine Perforation oder Lochung luftdurchlässig ausgeführt. Alternativ ist es möglich, die Folie 4 aus einem von Haus aus luftdurchlässigen Material auszubilden. Die Folie 4 ist zudem eingefärbt, so-dass sie im sichtbaren Spektrum bestimmte Wellenlängen absorbiert und andere durchlässt.

Zwischen der Folie 4, die nachfolgend auch als Abdeckfolie bezeichnet ist, und der Terrassenplatte 3 ist ein die Mikroorganismen enthaltendes Reservoir 6 angeordnet. Hierbei kann es sich um eine weitere Folie aus einem der vorstehend im Zusammenhang mit der Abdeckfolie 4 genannten Masterialien handeln. Die Mikroorganismen sind beispielsweise in Hohlräumen des Reservoirs 6 angeordnet. Die Mikroorganismen können auch als Beschichtung auf dem Reservoir 6 aufgebracht sein.

Zwischen dem Reservoir 6 und der Abdeckfolie 4 ist bei der Reinigungs-vorrichtung 1 nach Fig. 1 eine nährstoffhaltige Beschichtung 7, enthaltend Nährstoffe für die Mikroorganismen, angeordnet Die nährstoffhaltige Beschichtung 7 kann auf der Abdeckfolie 4 oder auf dem Reservoir 6 als Schicht aufgetragen sein. Es ist auch möglich, sowohl die nährstoffhaltige Beschichtung 7 als auch das Reservoir 6 als zwei Beschichtungen auf der Abdeckfolie 4 aufzubringen.

Zwischen dem Reservoir 6 und der nährstoffhaltigen Beschichtung 7 kann eine Trennwand oder Trennschicht vorgesehen sein, die durch gezielt beaufschlagte Einflüsse oder durch Umwelteinflüsse, beispielsweise durch Druck oder Temperatur, kollabiert und hierdurch eine Verbindung zwischen der nährstoffhaltigen Beschichtung 7 und dem Reservoir 6 zur Zufuhr von Nährstoffen zu den Mikroorganismen schafft.

Zum Reinigen der Terrassenplatte 3 wird die Abdeckfolie 4 zusammen mit der nährstoffhaltigen Beschichtung 7 und dem Reservoir 6 auf der Terrassenplatte 3 aufgebracht. Die Mikroorganismen bauen dann die Algen und das Moos 2 im Laufe der Zeit ab. Die Schichten 4, 6 und 7 der Habitat-Vorgabeeinrichtung können hinsichtlich ihrer biologischen Abbaubarkeit so abgestimmt sein, dass sie zu einem Zeitpunkt abgebaut sind, an dem eine vollständige Reinigung der Terrassenplatte 3 durch den Abbau der Algen und des Mooses 2 durch die Mikroorganismen zu erwarten ist. Der Abbau der Habitat-Vorgabeeinrichtung kann auch unter dem Einfluss beispielsweise von UV-Strahlung vonstatten gehen. Alternativ kann nach einer derartigen vorgegebenen Zeitspanne die nicht abgebaute Habitat-Vorgabeeinrichtung 4, 6 und 7 von der Terrassenplatte 3 abgenommen werden. Letztere liegt dann in gereinigtem Zustand vor. Der biologische Abbau der Verschmutzung auf der Terrassenplatte 3 kann, je nach Luft- und Licht-durchlässigkeit der Habitat-Vorgabeeinrichtung 4, 6, 7 auch so erfolgen, dass die biologisch abbaubare Verschmutzung auf der Terrassenplatte 3 zunächst abstirbt und anschließend von den Mikroorganismen abgebaut wird.

Die Reinigungsvorrichtung 1 muss das Reservoir 6 sowie die nährstoffhaltige Beschichtung 7 nicht zwingend enthalten. In diesem Fall besteht die Habitat-Vorgabeeinrichtung ausschließlich aus der Abdeckfolie 4. In vielen Fällen siedeln sich die zum biologischen Abbau der Algen und des Mooses 2 notwendigen Mikroorganismen ohne aktive Zugabe an. Bei einer weiteren Variante der Reinigungsvorrichtung 1 ist in der Habitat-Vorgabeeinrichtung ein Reservoir, jedoch keine nährstoffhaltige Beschichtung vorhanden. Schließlich ist es möglich, zwar eine nährstoffhaltige Beschichtung, aber kein Mikroorganismen-Reservoir bei der Habitat-Vorgabeeinrichtung vorzusehen.

Fig. 2 zeigt eine weitere Ausführung einer Reinigungsvorrichtung 1. Komponenten, die denjenigen entsprechen, die vorstehend unter Bezugnahme auf die Fig. 1 schon erläutert wurden, tragen die gleichen Bezugsziffern und werden nicht nochmals im Einzelnen diskutiert. Dies gilt auch für das Aufbringungsverfahren.

Die Habitat-Vorgabeeinrichtung 8 nach Fig. 2 umfasst eine Beschichtung in Form eines Leimfarben-Anstrichs 8. Der Anstrich 8 verwendet Leim als Bindemittel und Wasser als Lösungsmittel. Die Leimfarbe kann zusätzlich Pigmente und Füllstoffe wie Kalksteinmehl, Lithopone oder Kreide enthalten. Zwischen dem Anstrich 8 und der Terrassenplatte 3 können wiederum ein Reservoir sowie eine nährstoffhaltige Beschichtung vorgesehen sein, wie dies im Zusammenhang mit der Ausführung nach Fig. 1 schon erläutert wurde. Ein Mikroorganismen-Reservoir sowie eine nährstoffhaltige Beschichtung können beim Anstrich 8 auch dadurch erzeugt werden, dass entsprechend gebundene Mikroorganismen sowie Nährstoffe dem Anstrich 8 vor dem Auftrag beigegeben werden.

Alternativ zu einem Leimfarbenanstrich kann auch ein Lack- oder ein Farbanstrich zum Einsatz kommen. Die Eigenschaften des Anstrichs 8 in Bezug auf sein spektrales Verhalten im UV/VIS/IR und in Bezug auf seine Luftdurchlässigkeit werden entsprechend dem vorgegeben, was vorstehend zu Fig. 1 schon erläutert wurde.

Um ausreichend Raum für die Mikroorganismen zwischen dem Anstrich 8 und der Terrassenplatte 3 zu schaffen, kann die Viskosität des Anstrichs 8 entsprechen vorgegeben werden. Es ist auch möglich, in den Anstrich 8 vor dessen Auftrag gezielt Luft einzublasen, sodass beim aufgetragenen Anstrich 8 Luftbläschen vorhanden sind, in denen sich die Mikroorganis-men entwickeln können.

Der Anstrich 8 ist insbesondere abziehbar oder abdampfbar gestaltet.

Fig. 3 zeigt eine weitere Ausführung einer Reinigungsvorrichtung. Komponenten, die denjenigen entsprechen, die vorstehend unter Bezugnahme auf die Fig. 1 und 2 schon erläutert wurden, tragen die gleichen Bezugsziffern und werden nicht nochmals im Einzelnen diskutiert. Dies gilt auch für das Aufbringungsverfahren.

Die Habitat-Vorgabeeinrichtung umfasst bei der Ausführung nach Fig. 3 einen Schaum 9. Beim Schaum 9 kann es sich um ein Proteinschaummittel handeln. Beim Schaum 9 kann es sich um ein Fluorproteinschaummittel (FPS) oder um ein filmbildendes Fluorproteinschaummittel (FFFP) handeln. Die Eigenschaften des Anstrichs 8 in Bezug auf sein spektrales Verhalten im UV/VIS/IR und in Bezug auf seine Luftdurchlässigkeit werden entsprechend dem vorgegeben, was vorstehend zu Fig. 1 schon erläutert wurde.

Als Schaum 9 kann auch ein anderer Schwerschaum oder Mittelschaum zum Einsatz kommen. Je nach den strukturellen und elektrostatischen Eigenschaften der im Schaum 9 vorliegenden oberflächenaktiven Moleküle bilden sich Schaumbläschen mit unterschiedlicher Größe, Wandstärke und Lebensdauer, die auf die Anforderungen der eingesetzten Mikroorganismen hinsichtlich der Habitat-Bildung abgestimmt werden können. Eine Stabilisierung des Schaums 9 wird über den Marangoni-Effekt erreicht.

Der Schaum 9 ist insbesondere von der Terrassenplatte 3 abziehbar oder abdampfbar gestaltet.

Fig. 4 zeigt eine weitere Ausführung einer Reinigungsvorrichtung. Komponenten, die denjenigen entsprechen, die vorstehend unter Bezugnahme auf die Fig. 1 und 2 schon erläutert wurden, tragen die gleichen Bezugsziffern und werden nicht nochmals im Einzelnen diskutiert. Dies gilt auch für das Aufbringungsverfahren.

Eine Habitat-Vorgabeeinrichtung der Reinigungsvorrichtung 1 nach Fig. 4 umfasst eine Fasermaterial-Abdeckung 10. Hierbei kann es sich um ein Gewebe oder um ein nicht gewebtes Fasermaterial, also um ein Vlies, handeln. Als Fasermaterial-Abdeckung 10 können insbesondere Vliese aus Textilien, Baumwolle, Wolle, Papier, Filz, Polypropylen (PP), Polyester, Viskose oder Mischgewebe eingesetzt werden. Die Fasermaterial-Abdekkung kann, beispielsweise zur Schaffung eines Mikroorganismen-Reservoirs und/oder einer nährstoffhaltigen Beschichtung, entsprechend getränkt und/oder vorbehandelt werden.

Die Eigenschaften der Fasermaterial-Abdeckung 10 in Bezug auf ihr spektrales Verhalten im UV/VIS/IR und in Bezug auf ihre Luftdurchlässigkeit weiden entsprechend dem vorgegeben, was vorstehend zu Fig. 1 schon erläutert wurde.

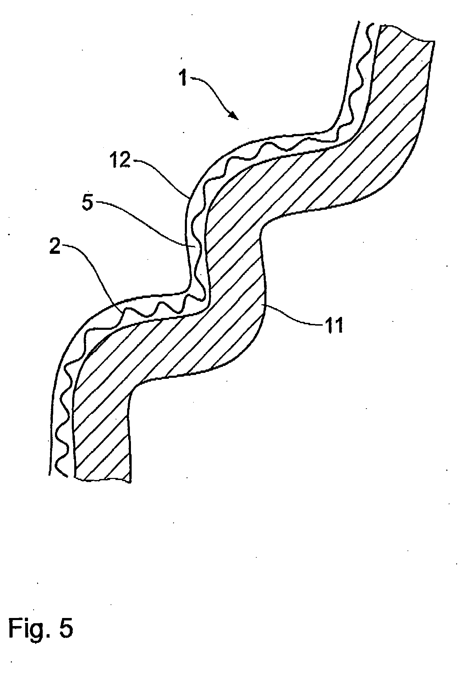

Fig. 5 zeigt eine weitere Ausführung einer Reinigungsvorrichtung 1 am Beispiel der Reinigung eines Zierelements 11 aus Stein als zu reinigendem Gegenstand. Komponenten, die denjenigen entsprechen, die vorstehend schon unter Bezugnahme auf die Fig. 1 bis 4 erläutert wurden, tragen die gleichen Bezugsziffern und werden nicht nochmals im Einzelnen diskutiert. Dies gilt auch für das Aufbringungsverfahren.

Bei der Ausführung nach Fig. 5 umfasst die Habitat-Vorgabeeinrichtung eine knetbare Abdeckmasse 12. Letztere kann nach Art eines Typengum-mis ausgeführt sein, der zur Reinigung der Typen von Schreibmaschinen verwendet wird. Die Abdeckmasse 12 kann wiederum ein Mikroorganismen-Reservoir und/oder Nährstoffe für die Mikroorganismen beinhalten.

Die Eigenschaften der Abdeckmasse 12 in Bezug auf ihr spektrales Verhalten im UV/VIS/IR und in Bezug auf ihre Luftdurchlässigkeit werden entsprechend dem vorgegeben, was vorstehend zu Fig. 1 schon erläutert wurde.

Die Abdeckmasse wird bei der Durchführung des Reinigungsverfahrens auf das Zierelement 11 auf dessen zu reinigende Bereiche aufgedrückt. Die Abdeckmasse passt sich auch in kleinste Vertiefungen und Rücksprünge des Zierelements 11 an, sodass die Mikroorganismen, die sich zwischen der Abdeckmasse 12 und im Zierelement 11 bilden, Verschmutzungen auch in derart unzugänglichen Abschnitte erreichen.

## Patentansprüche

1. Reinigungsvorrichtung (1) zur Reinigung eines insbesondere mit Algen oder Moos (2) befallenen flächigen Gegenstandes (3; 11), **gekennzeichnet durch** eine flächige Habitat-Vorgabeeinrichtung (4; 8; 9; 10; 12), welche zur Schaffung eines zwischen der Vorgabeeinrichtung (4; 8; 9; 10; 12) und dem zu reinigenden Gegenstand (3; 11) angeordneten Lebensraums (5) für Mikroorganismen auf den zu reinigenden Gegenstand (3; 11) diesen abdeckend aufbringbar ist.

2. Reinigungsvorrichtung nach Anspruch 1, **dadurch gekennzeichnet, dass** die Habitat-Vorgabeeinrichtung (4; 8; 9; 10; 12) aus wasserlöslichem Material ausgeführt ist.

3. Reinigungsvorrichtung nach Anspruch 1 oder 2, **dadurch gekennzeichnet, dass** die Habitat-Vorgabeeinrichtung (4; 8; 9; 10; 12) aus einem für UV-Strahlung zumindest teilweise absorbierenden Material ausgeführt ist.

4. Reinigungsvorrichtung nach einem der Ansprüche 1 bis 3, **dadurch gekennzeichnet, dass** die Habitat-Vorgabeeinrichtung (4; 8; 9; 10; 12) aus luftdurchlässigem Material ausgeführt ist.

5. Reinigungsvorrichtung nach einem der Ansprüche 1 bis 4, **dadurch gekennzeichnet, dass** die Habitat-Vorgabeeinrichtung (4; 8; 9; 10; 12) aus einem für IR-Strahlung zumindest teilweise absorbierenden Material ausgeführt ist.

6. Reinigungsvorrichtung nach einem der Ansprüche 1 bis 5, **gekennzeichnet durch** ein Mikroorganismen enthaltendes Reservoir (6), welches insbesondere mit der Habitat-Vorgabeeinrichtung (4; 8; 9; 10; 12) zu einem vorgegebenen Zeitpunk in Verbindung gebracht werden kann.

7. Reinigungsvorrichtung nach einem der Ansprüche 1 bis 6, **gekennzeichnet durch** eine nährstoffhaltige Habitat-Vorgabeeinrichtung (4; 8; 9; 10; 12).

8. Reinigungsvorrichtung nach Anspruch 7, **gekennzeichnet durch** eine nährstoffhaltige Beschichtung (7) eines Grundkörpers (4; 10) der Habitat-Vorgabeeinrichtung.

9. Reinigungsvorrichtung nach einem der Ansprüche 1 bis 8, **dadurch gekennzeichnet, dass** die Habitat-Vorgabeeinrichtung eine Abdeckfolie (4) aufweist.

10. Reinigungsvorrichtung nach einem der Ansprüche 1 bis 8, **dadurch gekennzeichnet, dass** die Habitat-Vorgabeeinrichtung eine am zu reinigenden Gegenstand (3; 11) zumindest zeitweilig anhaftbare Beschichtung (8; 9; 12) aufweist.

11. Reinigungsvorrichtung nach Anspruch 10, **dadurch gekennzeichnet, dass** die Habitat-Vorgabeeinrichtung als Anstrichmittel (8) ausgeführt ist.

12. Reinigungsvorrichtung nach einem der Ansprüche 1 bis 8, **dadurch gekennzeichnet, dass** die Habitat-Vorgabeeinrichtung eine Fasermaterial-Abdeckung (10) aufweist.

13. Reinigungsvorrichtung nach Anspruch 10, **dadurch gekennzeichnet, dass** die Habitat-Vorgabeeinrichtung als knetbare Abdeckmasse (12) ausgeführt ist.

14. Verfahren zur Reinigung eines flächigen Gegenstandes (3; 11) mit einer Reinigungseinrichtung (1) nach einem der Ansprüche 1 bis 14 mit folgendem Schritt:
- Aufbringen der Habitat-Vorgabeeinrichtung (4; 8; 9; 10; 12) auf den Gegenstand (3; 11) derart, dass zwischen der Vorgabeeinrichtung (4; 8; 9; 10; 12) und dem zu reinigenden Gegenstand (3; 11) ein Lebensraum für Mikroorganismen gebildet wird.

15. Verfahren nach Anspruch 14 unter Verwendung einer Reinigungsvorrichtung nach Anspruch 6, **dadurch gekennzeichnet, dass** Mikroorganismen aus dem Reservoir (6) der Habitat-Vorgabeeinrichtung (4; 8; 9; 10; 12) zugegeben werden.
